(19) Europäisches Patentamt
European Patent Office
Office européen des brevets

(11) Veröffentlichungsnummer: **0 012 983**
**B1**

# (12) EUROPÄISCHE PATENTSCHRIFT

(45) Veröffentlichungstag der Patentschrift: **31.03.82**

(51) Int. Cl.³: **C 07 C 127/19,** C 07 D 235/26

(21) Anmeldenummer: **79105242.6**

(22) Anmeldetag: **18.12.79**

(54) **Diaminophenylharnstoffe, Verfahren zu ihrer Herstellung und ihre Verwendung zur Herstellung von Aminobenzimidazolonen-(2).**

(30) Priorität: **23.12.78 DE 2855883**

(43) Veröffentlichungstag der Anmeldung: **09.07.80 Patentblatt 80/14**

(45) Bekanntmachung des Hinweises auf die Patenterteilung: **31.03.82 Patentblatt 82/13**

(84) Benannte Vertragsstaaten: **BE CH DE FR GB IT**

(56) Entgegenhaltungen: **keine**

(73) Patentinhaber: **HOECHST AKTIENGESELLSCHAFT, Postfach 80 03 20, D-6230 Frankfurt/Main 80 (DE)**

(72) Erfinder: **Arndt, Otto, Dr., Frankfurter Strasse 38, D-6238 Hofheim am Taunus (DE)**
Erfinder: **Papenfuhs, Theodor, Dr., Heinrich-Bleicher-Strasse 40, D-6000 Frankfurt am Main 50 (DE)**
Erfinder: **Böhme, Peter, Dr., Am Flachsland 56, D-6233 Kelkheim (Taunus) (DE)**
Erfinder: **Tronich, Wolfgang, Dr., Martin-Wohmann-Strasse 27, D-6238 Hofheim am Taunus (DE)**
Erfinder: **Mees, Bernhard, Dr., Gräfliche Strasse 31, D-6239 Eppstein/Taunus (DE)**

EP 0 012 983 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Diaminophenylharnstoffe, Verfahren zu ihrer Herstellung und ihre Verwendung zur Herstellung von Aminobenzimidazolonen-(2)

5-Aminobenzimidazolone-(2) haben als Zwischenprodukte zur Herstellung von Pigmenten Bedeutung erlangt.

Zur Herstellung der 5-Aminobenzimidazolone-(2) sind mehrere Verfahren bekannt geworden:

So ist bereits in Chem. Ber. 17, (1884), 2631 die Reduktion von N-(2,4-Dinitrophenyl)-äthyl-urethan zu N-(2,4-Diaminophenyl)-äthyl-urethan und dessen Ringschlußreaktion unter Alkoholabspaltung zum 5-Aminobenzimidazolon-(2) beschrieben. Dieses Verfahren hat jedoch wegen der Schwerzugänglichkeit des reinen Ausgangsprodukts und unbefriedigender Ausbeute und Reinheit des Endprodukts keine praktische Bedeutung erlangt.

Aus Monatshefte f. Chemie 107, (1976) 1307-10 ist die Nitrierung von Benzimidazolon-(2) zu 5-Nitrobenzimidazolon-(2) bekannt. Dieses ist durch Reduktion der Nitrogruppe in 5-Aminobenzimidazolon-(2) überführbar. Ein Nachteil dieses Verfahrens besteht darin, daß die Bildung von Isomeren sowie höher nitrierten Benzimidazolonen-(2) nicht völlig verhindert werden kann. Außerdem stellt die Nitrierung eine zusätzliche Verfahrensstufe mit entsprechendem technischen Aufwand dar.

Ein prinzipiell anderer Weg zur Herstellung von 5-Nitrobenzimidazolon-(2) wird in J. Chem. Soc. C (1971) 1139 mit der Thermolyse von 2,4-Dinitrophenylglycin beschritten. Jedoch ist dieses Verfahren wegen seiner mäßigen Ausbeute und der Bildung beträchtlicher Mengen unbekannter Zersetzungsprodukte von keinerlei praktischem Interesse.

Schließlich ist aus der DE-OS 2 725 957 bekannt, daß man 1,2-Diamino-4-nitro-benzol in organischen Medien mit Harnstoff zu 5-Nitrobenzimidazolon-(2) kondensieren und dieses, z. B. mit Eisen, zum 5-Aminobenzimidazolon-(2) reduzieren kann. Die Herstellung von 1,2-Diamino-4-nitrobenzol ist jedoch aufgrund der partiellen Reduktion einer Nitrogruppe in 2,4-Dinitroanilin mit Natriumsulfid sehr aufwendig und problematisch und daher ebenfalls nicht von praktischem Interesse.

Die Ringschlußreaktion von 2-Amino-phenylharnstoff zu Benzimidazolon ist aus Gazz. Chim. Ital. 491 (1919) 22 und von 2-Amino-4-bzw.-5-carboxyphenylharnstoff zu Benzimidazolon-5-carbonsäure aus Ann. 291, 327 (1896) bekannt. Die Herstellung der erwünschten 5-Aminobenzimidazolone führt jedoch ebenfalls über die Nitrierung der Benzimidazolone und anschließende Reduktion der Nitrobenzimidazolone, was wiederum mit zwei zusätzlichen Arbeitsgängen und den bereits erwähnten Problemen verbunden ist. Bemerkenswert ist, daß bei der beschriebenen Synthese zunächst eine Dinitroverbindung hergestellt wird, aus der jedoch eine Nitrogruppe vor der Reduktion abgespalten wird.

Die Ringschlußreaktion geeigneter Diaminophenylharnstoffe, die auf einfache Weise und unmittelbar zu den gewünschten Aminobenzimidazolonen führen sollte, ist somit in der Literatur bisher nicht beschrieben worden.

Gegenstand der Erfindung sind Verbindungen der allgemeinen Formel

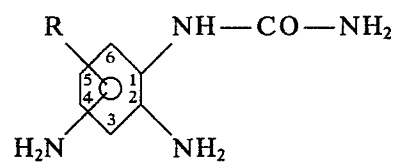

in der R Wasserstoff, Halogen, niederes Alkyl, niederes Alkoxy, Phenyl, Phenoxy oder einen ankondensierten Benzolring bedeutet, ein Verfahren zur Herstellung dieser Verbindungen, das dadurch gekennzeichnet ist, daß man einen Dinitrophenylharnstoff der allgemeinen Formel

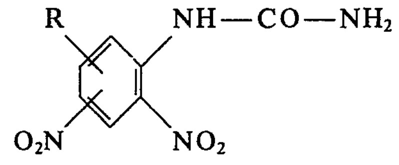

in der R die vorstehend genannte Bedeutung hat, reduziert, sowie die Verwendung der erfindungsgemäßen Verbindungen zur Herstellung von Aminobenzimidazolonen der Formel

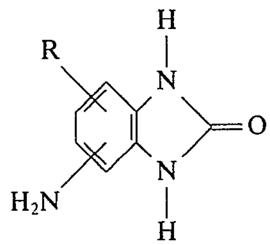

in der R die vorstehend genannte Bedeutung hat, durch Ringschluß unter Ammoniakabspaltung, durch Erhitzen in einem flüssigen Medium.

Die als Ausgangsmaterialien eingesetzten Dinitrophenylharnstoffe sind aus der US-PS 28 26 611 und aus J. pr. Ch. 110 (1925) 300 bekannt und nach den dort beschriebenen Verfahren zugänglich.

Die Erfindung kann somit durch das allgemeine Reaktionsschema wiedergegeben werden:

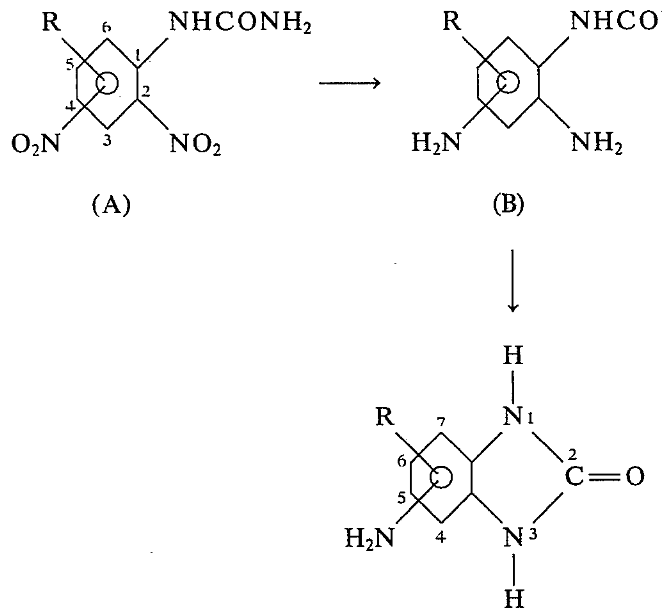

(A) (B)

(C)

**Hierbei stellt R die genannten inerten Substituenten dar, vorzugsweise Wasserstoff, Chlor, Methyl, Methoxy oder einen ankondensierten Benzolring, wobei die zweite Aminogruppe in 6- oder 4-Stellung steht, insbesondere jedoch einen Substituenten mit der in Tabelle I angegebenen Bedeutung:**

Tabelle I

| R | Stellung der Nitrogruppe in der Formel A |
|---|---|
| H | 6 |
| H | 4 |
| 6-$CH_3$ | 4 |
| 6-Cl | 4 |
| 5-$CH_3$ | 4 |
| 5-$OCH_3$ | 4 |
| in 5- und 6-Stellung ankondensierter Benzolring | 4 |

Prinzipiell können die nach der Reduktion der Dinitrophenylharnstoffe erhaltenen Diaminophenylharnstoffe isoliert und in Abwesenheit des Reduktionsmittels — und bei einer katalytisch durchgeführten Reduktion auch in Abwesenheit des Katalysators — ringgeschlossen werden. Eine bevorzugte Ausführungsform des Verfahrens besteht jedoch darin, daß die Ringschlußreaktion ohne Zwischenisolierung des Diaminophenylharnstoffs ausgeführt wird.

Die Reduktion der Nitrogruppen kann nach literaturbekannten Verfahren durchgeführt werden, z. B. mit Eisen, bevorzugt kommt jedoch die katalytische Hydrierung zur Anwendung.

Als Reduktionsmedium bzw. Verdünnungsmittel können alle in solchen Fällen allgemein verwendeten organische oder wäßrige Medien oder deren Gemische verwendet werden; eine besonders einfache, kostengünstige und damit bevorzugte Ausgestaltungsform besteht in der Verwendung von Wasser als Reduktions- und Ringschlußmedium. Der pH-Wert richtet sich bei der katalytischen Reduktion insbesondere nach der Art des verwendeten Katalysators, z. B. nach dessen Säure- bzw. Alkalibeständigkeit. In einer bevorzugten Ausgestaltungsform der katalytischen Reduktion kommen z. B. Nicht-Edelmetallkatalysatoren, insbesondere Nickelkatalysatoren zum Einsatz, wozu der pH-Wert des Reduktionsmediums gegebenenfalls durch Zusatz geeigneter Puffersubstanzen so eingestellt wird, daß er nicht unterhalb 6 absinkt und insbesondere beim Ringschluß zum Aminobenzimidazolon-(2) nicht wesentlich über 10 ansteigt. Bevorzugt wird die katalytische Reduktion in Gegenwart eines Phosphat-Puffersystems bei pH 6 bis 7 durchgeführt; während des Ringschlusses steigt der pH-Wert durch das freigesetzte Ammoniak auf einen Wert von 8 bis 10 an.

Der Ringschluß kann jedoch auch bei einem pH-Wert kleiner 8, bevorzugt kleiner 6, durchgeführt werden, wenn man dafür sorgt, daß das entstehende Ammoniak mittels Säure gebunden wird, wobei der pH bis etwa 1 absinken kann.

Die katalytische Reduktion kann selbstverständlich auch in Gegenwart von Edelmetallkatalysatoren, z. B. Palladium- oder Platin-Katalysatoren durchgeführt werden, wobei der pH-Wert der Reduktionsmischung auch unterhalb 6 liegen kann.

Wasserstoffdruck, Reduktionstemperatur und Reduktionszeit liegen im Bereich der für die katalytische Reduktion aromatischer Nitroverbindungen üblichen Bedingungen.

Der Ringschluß der Diaminophenylharnstoff-Verbindungen kann in Gegenwart oder Abwesenheit von Reduktionsmittel und Katalysator erfolgen. Eine besonders einfache und bevorzugte Ausführungsform des Verfahrens besteht z. B. darin, den Ringschluß unmittelbar nach der katalytischen Hydrierung der Dinitrophenylharnstoffe im Hydrierautoklaven auszuführen, wobei man vorher lediglich den Wasserstoff, z. B. durch Spülen mit Stickstoff, aus dem Gasraum entfernen kann, den Hydrierkatalysator jedoch im Autoklaven beläßt. Der Ringschluß kann drucklos bei Temperaturen bis zu 100°C oder — bevorzugt — bei Temperaturen über 100°C bei entsprechendem Überdruck erfolgen.

Die Dauer der Ringschlußreaktion richtet sich nach der jeweiligen Reaktionstemperatur und beträgt zwischen 3 und 10 Stunden. Der pH-Wert der Reaktionsmischung entspricht zu Beginn der Reaktion dem pH der Reduktionsmischung und steigt mit Fortgang der Ringschlußreaktion durch das freigesetzte Ammoniak je nach der Menge des beigesetzten Puffers bis 10 an.

Die Aufarbeitung des Reaktionsgemisches kann z. B. dadurch erfolgen, daß aus der erhaltenen alkalischen Lösung, gegebenenfalls nach Zusatz von wäßrigem Alkali, zunächst der Hydrierkontakt abgetrennt wird und anschließend das Aminobenzimidazolon-(2) durch Säurezugabe ausgefällt wird. Jedoch sind auch andere Aufarbeitungsmöglichkeiten denkbar.

Im folgenden ist eine besonders bevorzugte Ausgestaltungsform des Verfahrens näher beschrieben:

Der technisch feuchte Dinitro-aryl-harnstoff bzw. seine in Tabelle I beschriebenen Derivate werden in Wasser eingetragen, dem zweckmäßigerweise ein auf pH 6—7 abgestimmter Puffer (z. B. Phosphatpuffer) und etwas Aktiv-Kohle zugesetzt sind. Der Hydrierkatalysator, beispielsweise Nickel auf Kieselgur, wird zugesetzt. Dann wird nacheinander mit Stickstoff und Wasserstoff gespült und unter einem Druck von 40 bar Wasserstoff bis zum Beginn der Wasserstoffaufnahme auf ca. 35—50°C geheizt. Die Reduktion zu den Diaminoarylharnstoffen erfolgt im allgemeinen bei ca. 50—80°C, insbesondere bei 55—60°C unter einem Druck von 40 bar Wasserstoff. Nach Beendigung der Wasserstoffaufnahme wird der Wasserstoff durch Stickstoff verdrängt. Für die Ringschlußreaktion zu den Aminobenzimidazolonen-(2) bringt man die Reduktionslösung auf 100 bis 150°C. Sie ist je nach Reaktionstemperatur nach 2 bis 10 Stunden beendet. Durch die Ammoniakabspaltung wird das Reaktionsgemisch alkalisch (bis pH 10). Das Reaktionsgemisch kann in verschiedener Weise aufgearbeitet werden, je nachdem, ob zur Weiterverarbeitung katalysatorfreie Aminobenzimidazolone-(2) benötigt werden, oder ob katalysatorhaltige Produkte verwendet werden können.

So können zunächst nach Neutralisierung, beispielsweise mit Salzsäure, auf pH 7 die katalysatorhaltigen Aminobenzimidazolone-(2) von der Mutterlauge abgetrennt werden und in einem organischen Lösungsmittel, wie z. B. N-Methylpyrrolidon, oder verdünnter wäßriger Natronlauge oder in Salzsäure gelöst und dann vom Katalysator abfiltriert werden.

Die Lösungen im organischen Lösemittel eignen sich z. B. für die sofortige Weiterverarbeitung zu Folgeprodukten. Man kann aber auch die wäßrigen alkalischen Reaktionsmischungen, bevorzugt in

der Wärme, direkt vom Katalysator abfiltrieren und dann aus dem Filtrat die Aminobenzimidazolone-(2) durch Neutralisation fällen und nach eventueller Aufkonzentrierung, z. B. durch Filtration, isolieren.

Die nach dem erfindungsgemäßen Verfahren erhaltenen Aminobenzimidazolone-(2) sind wertvolle Zwischenprodukte für die Herstellung von Pigmenten.

In den folgenden Beispielen beziehen sich Teile und Prozentangaben auf das Gewicht, sofern nichts anderes angegeben ist.

## Beispiele

1.) 5-Amino-benzimidazolon-(2).
113 Teile 2,4-Dinitrophenylharnstoff werden in 3 l Wasser eingetragen, dem zur Abpufferung auf pH 6 8 Teile Phosphorsäure und 8 Teile 33%ige Natronlauge zugesetzt sind. Dann werden 10 Teile Aktivkohle und 4 Teile Nickelkatalysator (55% Ni auf Kieselgur) eingetragen. Die Suspension wird bei 40 bis 60°C hydriert und anschließend 8 Stunden auf 120°C erhitzt. Das abgekühlte Reaktionsgemisch wird mit 60 Teilen 33%iger Natronlauge versetzt und heiß in vorgelegte 100 Teile 30%ige Salzsäure filtriert. Das Filtrat wird im Vakuum auf 500 Teile eingeengt, das 5-Aminobenzimidazolon-(2) abgesaugt, mit Eiswasser gewaschen und im Vakuum bei 120°C unter Stickstoff getrocknet. Man erhält 64 Teile 5-Aminobenzimidazolon-(2), Fp. 234—236°C.

2.) 5-Amino-benzimidazolon-(2).
Im 5-l-Autoklaven werden vorgelegt: 3000 Teile Wasser, 16 Teile 85%ige Phosphorsäure, 16 Teile 33%ige Natronlauge, 20 Teile Aktivkohle, 227 Teile 2,4-Dinitrophenylharnstoff (1 Mol) und 8 Teile Nickelkatalysator auf Kieselgur. Die Suspension wird bei 30 bis 60°C im Autoklaven hydriert. Die erhaltene Reduktionslösung wird mit 15 Teilen Natriumdithionit versetzt und vom Katalysator geklärt. Das Filtrat läuft zu 265 Teilen 30%iger Salzsäure und wird anschließend 7 Stunden unter Rückfluß erhitzt. Man versetzt anschließend mit 136 Teilen 33%iger Natronlauge und isoliert das 5-Aminobenzimidazolon-(2) bei 5°C durch Filtration. Nach dem Trocknen im Vakuum erhält man 121 Teile 5-Aminobenzimidazolon-(2).

3.) 5-Amino-benzimidazolon-(2).
Im 5-l-Autoklaven werden vorgelegt:
3000 Teile Wasser, 16 Teile 85%ige Phosphorsäure, 16 Teile 33%ige Natronlauge, 20 Teile Aktivkohle, 227 Teile 2,4-Dinitrophenylharnstoff und 1 Teil Katalysator (5% Palladium auf Kohle). Die Suspension wird bei 30 bis 60°C im Autoklaven hydriert. Die erhaltene Reduktionslösung wird mit 30 Teilen Natriumdithionit versetzt, auf 265 Teile 30%ige Salzsäure gegeben und 6 Stunden unter Rückfluß erhitzt. Dann wird heiß vom Katalysator geklärt und mit 110 Teilen 33%iger Natronlauge versetzt. Das beim Abkühlen auskristallisierende 5-Aminobenzimidazolon-(2) wird bei 5°C durch Filtration isoliert. Nach dem Trocknen im Vakuum erhält man 121 Teile 5-Aminobenzimidazolon-(2).

4.) 2,4-Diaminophenylharnstoff.
Man verfährt bei der Reduktion wie in Beispiel 2, isoliert jedoch den 2,4-Diaminophenylharnstoff als Dihydrochlorid. Zu diesem Zweck klärt man die mit 15 Teilen Natriumdithionit versetzte Reduktionslösung vom Katalysator und läßt das Filtrat zu 575 Teilen 30%iger Salzsäure laufen. Die salzsaure Lösung des 2,4-Diaminophenylharnstoffs wird im Vakuum der Wasserstrahlpumpe bei max. 50°C bis auf 650 Teile einer weißen Suspension eingeengt und mit 290 Teilen 30%iger Salzsäure versetzt. Das auskristallisierte 2,4-Diaminophenylharnstoff-Dihydrochlorid wird bei 5°C durch Filtration isoliert und mit 400 Teilen 5°C kalter, 15%iger Salzsäure gewaschen.
Man erhält nach dem Trocknen im Vakuum bei 50°C 197 Teile 2,4-Diaminophenylharnstoff-Dihydrochlorid in Form weißer Kristalle. Aus der Mutterlauge läßt sich neben geringen Mengen 5-Aminobenzimidazolon-(2) weiteres 2,4-Diaminophenylharnstoff-Dihydrochlorid isolieren. Das 2,4-Diaminophenylharnstoff-Dihydrochlorid läßt sich in wäßriger Lösung, wie in Beispiel 2 beschrieben, zum 5-Aminobenzimidazolon-(2) ringschließen.

5.) 5-Amino-benzimidazolon-(2).
100 Teile Nadeleisenschleifstaub und 100 Teile Eisenspäne werden mit 800 Teile Wasser in Gegenwart von 30 Teilen Essigsäure 1 Stunde auf 90—100°C erhitzt. Dann werden in 1 Stunde bei 95°C 113 Teile 2,4-Dinitrophenylharnstoff (0,5 Mol) eingetragen und 2 Stunden bei 95°C unter Stickstoff nachgerührt. Man stellt bei 90°C mit 50 Teilen wasserfreiem Natriumcarbonat auf pH 8,5 und klärt vom Eisenoxid-Schlamm durch eine Drucknutsche. Der Eisenoxid-Schlamm wird mit 1000 Teilen heißem Wasser in Portionen gewaschen. Das Filtrat wird mit 120 Teilen 30%iger Salzsäure auf pH 4,0 gestellt und 10 Stunden unter Rückfluß gekocht (pH 5,0). Dann wird abgekühlt und mit 41 Teilen 33%iger Natronlauge auf pH 7,0 gestellt. Das auskristallisierte 5-Amino-benzimidazolon-(2) wird bei 5°C durch Filtration isoliert und mit Eiswasser gewaschen. Man erhält nach dem Trocknen im Vakuum bei 120°C 60 Teile 5-Amino-benzimidazolon-(2).

6.) 7-Chlor-5-amino-benzimidazolon-(2).
Im 5-l-Autoklaven werden vorgelegt:

3000 Teile Wasser, 23 Teile 85%ige Phosphorsäure, 39 Teile 33%ige Natronlauge, 40 Teile Aktivkohle, 428 Teile 6-Chlor-2,4-dinitrophenylharnstoff (1,64 Mol) und 5 Teile Nickelkatalysator auf Kieselgur. Die Reduktion erfolgt wie in Beispiel 1. Nach dem Entspannen des Wasserstoffs wird unter Rühren auf 150° C geheizt und 3 Stunden bei dieser Temperatur nachgerührt. Nach dem Abkühlen der Reaktionslösung wird das schwerlösliche 7-Chlor-5-amino-benzimidazolon-(2) zusammen mit dem Katalysator abfiltriert und mit 2000 Teilen Wasser gewaschen. Das 7-Chlor-5-amino-benzimidazolon-(2) wird aus dem Gemisch mit Kohle und Katalysator durch Behandeln mit 5 l Wasser und 230 Teilen 30%iger Salzsäure bei 50° C herausgelöst und die Lösung von der Kohle und dem Katalysator geklärt. Aus dem Filtrat wird das 7-Chlor-5-amino-benzimidazolon-(2) mit 450 Teilen 25%igem Ammoniak bei pH 9 gefällt. Das Produkt wird bei 50° C durch Filtration isoliert und mit 2000 Teilen Wasser gewaschen. Man erhält 267 Teile 7-Chlor-5-amino-benzimidazolon-(2) vom Fp. 274—275° C.

7.) 7-Methyl-5-amino-benzimidazolon-(2).
Im 1-l-Autoklav werden vorgelegt:
300 Teile Trinkwasser, 1,6 Teile 85%ige Phosphorsäure, 1,7 Teile 33%ige Natronlauge, 2 Teile Aktivkohle, 25 Teile 6-Methyl-2,4-dinitrophenylharnstoff (0,10 Mol) und 1,50 Teile Nickelkatalysator auf Kieselgur. Die Reduktion erfolgt wie in Beispiel 1.
Nach dem Entspannen des Wasserstoffs wird unter Rühren auf 120° C geheizt und 8 Stunden bei dieser Temperatur nachgerührt. Die Reaktionslösung wird mit 2 Teilen Natriumdithionit versetzt, mit 100 Teilen Wasser verdünnt, mit 25 Teilen 33%iger Natronlauge (0,2 Mol) versetzt und bei 90° C unter Stickstoff vom Katalysator geklärt. Der Katalysator wird mit 50 Teilen heißer 5%iger Natronlauge, dann mit heißem Wasser gewaschen. Das Filtrat läuft bei der Klärung direkt in 44 Teile 30%ige Salzsäure. Aus dem Filtrat kristallisiert das 7-Methyl-5-amino-benzimidazolon-(2) aus. Es wird abfiltriert und mit Wasser gewaschen. Man erhält nach dem Trocknen im Vakuum bei 120° C 12,5 Teile 7-Methyl-5-amino-benzimidazolon-(2) vom Fp. 308—309° C.

8.) 6-Methyl-5-amino-benzimidazolon-(2).
Ansatzgröße und Versuchsdurchführung entsprechen dem Beispiel 7, jedoch mit dem Unterschied, daß man an Stelle von 6-Methyl-2,4-dinitro-phenylharnstoff 25 Teile 5-Methyl-2,4-dinitrophenylharnstoff einsetzt. Man erhält nach dem Trocknen im Vakuum bei 120° C 11 Teile 6-Methyl-5-amino-benzimidazolon-(2); Fp. 301—303° C.

9.) 6-Methoxy-5-amino-benzimidazolon-(2).
Im 2-l-Autoklav werden vorgelegt:
1000 Teile Wasser, 8 Teile 85%ige Phosphorsäure, 13,6 Teile 33%ige Natronlauge, 10 Teile Aktivkohle, 128 Teile 5-Methoxy-2,4-dinitrophenylharnstoff (0,5 Mol) und 4 Teile Nickelkatalysator auf Kieselgur. Die Reduktion erfolgt wie in Beispiel 1. Der Wasserstoff wurde nicht entspannt. Die Lösung wird auf 150° C geheizt und 3 Stunden bei dieser Temperatur gehalten. Die abgekühlte Reaktionslösung wird mit 10 Teilen Natriumdithionit versetzt und mit 35 Teilen 30%iger Salzsäure auf pH 7,0 gestellt. Dann wird mit 2000 Teilen heißem Wasser verdünnt und bei 90° C unter Stickstoff vom Katalysator geklärt. Das Filtrat wird im Vakuum der Wasserstrahlpumpe auf ca. 500 Teile eingeengt. Das auskristallisierte 6-Methoxy-5-aminobenzimidazolon-(2) wird bei 5° C durch Filtration isoliert und mit 150 Teilen Eiswasser gewaschen. Man erhält nach dem Trocknen im Vakuum bei 120° C 62 Teile 6-Methoxy-5-amino-benzimidazolon-(2), Fp. 248—249° C.

10.) 5-Amino-1-H-2-naphth[1,2-d]-imidazolon.
Im 2-l-Autoklav werden vorgelegt:
750 Teile Wasser, 4 Teile 85%ige Phosphorsäure, 6,8 Teile 33%ige Natronlauge, 5 Teile Aktivkohle, 57 Teile 2,4-Dinitro-naphthyl-harnstoff (0,2 Mol) und 3 Teile Nickelkatalysator auf Kieselgur. Die Reduktion erfolgt wie in Beispiel 1. Nach dem Entspannen des Wasserstoffs wird unter Rühren auf 150° C geheizt und 3 Stunden bei dieser Temperatur nachgerührt. Die Suspension wird auf 25° C abgekühlt, mit 16 Teilen 30%iger Salzsäure auf pH 7,0 gestellt und filtriert. Das schwerlösliche 5-Amino-naphthimidazolon-(2) wird in 3000 Teilen Wasser und 100 Teilen 33%iger Natronlauge bei 90° C gelöst und vom Katalysator geklärt. Der Katalysator wird mit 100 Teilen heißer 5%iger Natronlauge gewaschen. Das Filtrat wird mit 115 Teilen 30%iger Salzsäure auf pH 7,0 gestellt, das gefällte 5-Amino-naphthimidazolon-(2) wird bei 25° C abfiltriert und mit Wasser gewaschen. Man erhält nach dem Trocknen bei 120° C im Vakuum 26 Teile 5-Amino-naphthimidazolon-(2), Fp. 295—296° C.

11.) 4-Amino-benzimidazolon-(2).
Im 2-l-Autoklav werden vorgelegt:
600 Teile Wasser, 3,2 Teile 85%ige Phosphorsäure, 3,3 Teile 33%ige Natronlauge, 4 Teile Aktivkohle, 46 Teile 2,6-Dinitrophenylharnstoff (0,2 Mol) und 3 Teile Nickelkatalysator auf Kieselgur. Die Reduktion erfolgt wie in Beispiel 1. Nach dem Entspannen des Wasserstoffs wird unter Rühren auf 120° C geheizt und 8 Stunden bei dieser Temperatur nachgerührt. Die Reaktionslösung wird mit 5 Teilen Natriumdithionit und mit 25 Teilen 33%iger Natronlauge versetzt. Dann wird bei 90° C unter Stickstoff vom Katalysator geklärt. Der Katalysator wird mit 100 Teilen heißer 5%iger Natronlauge, dann mit heißem Wasser gewaschen. Das Filtrat läuft bei der Klärung direkt zu 44 Teilen 30%iger Salzsäure. Das Filtrat wird auf ein Drittel im Vakuum der

Wasserstrahlpumpe eingeengt. Das auskristallisierte 4-Amino-benzimidazol-(2) wird bei 5°C durch Filtration isoliert und mit Eiswasser gewaschen. Man erhält nach dem Trocknen im Vakuum bei 120°C 23 Teile 4-Amino-benzimidazolon-(2), Fp. 299—300°C.

Kernresonanzspektrum des 2,4-Diaminophenylharnstoff-Dihydrochlorids (Beispiel 4):

$\delta$ (DMSO $d_6$):

8,5—9,5 ppm (breite Signale, 9 H, N—H)
7,55 ppm (d, 1 H, H—6)
7,43 ppm (d, 1 H, H—3)
7,23 ppm (dd, 1 H, H—5)

Schmelzpunkt: 184—187°C

**Patentansprüche**

1. Verbindungen der allgemeinen Formel

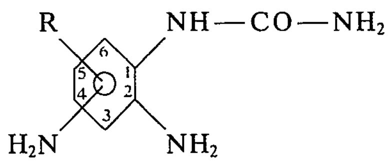

in der R Wasserstoff, Halogen, niederes Alkyl, niederes Alkoxy, Phenyl, Phenoxy oder einen ankondensierten Benzolring bedeutet.

2. Verbindungen nach Anspruch 1, dadurch gekennzeichnet, daß R Wasserstoff, Chlor, Methyl, Methoxy oder einen ankondensierten Benzolring bedeutet und die zweite Aminogruppe in 6- oder 4-Stellung steht.

3. Verfahren zur Herstellung der Verbindungen nach Anspruch 1, dadurch gekennzeichnet, daß man einen Dinitrophenylharnstoff der allgemeinen Formel

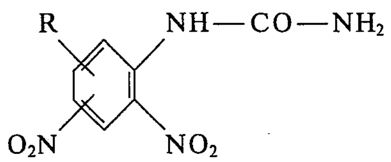

in der R die in Anspruch 1 genannte Bedeutung hat, reduziert.

4. Verfahren nach Anspruch 3, dadurch gekennzeichnet, daß die Reduktion eine katalytische Hydrierung ist.

5. Verfahren nach Anspruch 3 und 4, dadurch gekennzeichnet, daß die Reduktion im wäßrigen Medium erfolgt.

6. Verfahren nach Anspruch 4 und 5, dadurch gekennzeichnet, daß die Reduktion bei einem pH-Wert von mindestens 6 durchgeführt wird.

7. Verwendung der Verbindungen nach Anspruch 1 zur Herstellung von Aminobenzimidazolonen der Formel

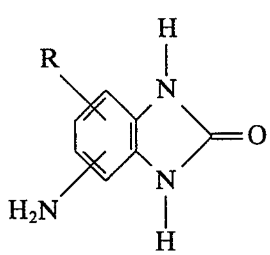

in der R die in Anspruch 1 genannte Bedeutung hat, durch Ringschluß unter Ammoniakabspaltung.

8. Verwendung nach Anspruch 7, dadurch gekennzeichnet, daß der Ringschluß im wäßrigen Medium erfolgt.

9. Verwendung nach Anspruch 7 und 8, dadurch gekennzeichnet, daß der Ringschluß bei einem pH-Wert von 1 bis 10 erfolgt.

10. Verwendung nach Anspruch 7 bis 9, dadurch gekennzeichnet, daß der Ringschluß mit einem nach Anspruch 3 erhaltenen Produkt ohne Zwischenisolierung erfolgt.

## Claims

1. Compounds of the general formula

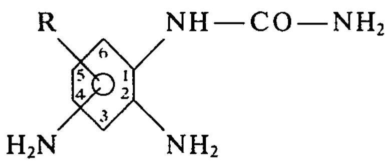

in which R is hydrogen, halogen, lower alkyl, lower alkoxy, phenyl, phenoxy or a fused benzene ring.

2. Compound according to claim 1, characterized in that R is hydrogen, chlorine, methyl, methoxy or a fused benzene ring, and the second amino group stands in 6- or 4-position.

3. Process for the preparation of the compounds according to claim 1, characterized by reducing a dimitrophenyl urea of the general formula

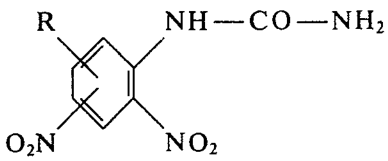

in which R has the meaning stated in claim 1.

4. Process according to claim 3, characterized in that the reduction is a catalytic hydrogenation.

5. Process according to claim 3 and 4, characterized in that the reduction occurs in an aqueous medium.

6. Process according to claims 4 and 5, characterized in that the reduction is performed at a pH-value of at least 6.

7. Use of the compounds according to claim 1 for the preparation of aminobenzimidazolones of the formula

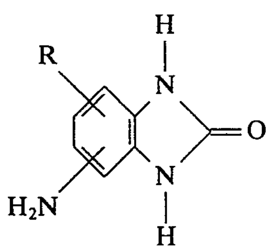

in which R has the meaning stated in claim 1, by ring closure with splitting off ammonia.

8. Use according to claim 7, characterized in that the ring closure occurs in an aqueous medium.

9. Use according to claims 7 and 8, characterized in that the ring closure occurs at a pH-value of 1 to 10.

10. Use according to claim 7 to 9, characterized in that the ring closure occurs with a product obtained according to claim 3 without intermediate isolation.

## Revendications

1. Les composés de formule générale

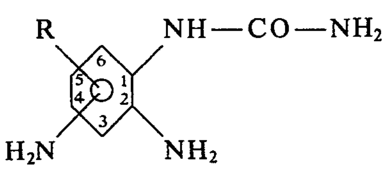

dans laquelle le symbole R représente l'hydrogène ou un halogène, un alkyle inférieur ou un alcoxy inférieur, un phényle ou un phénoxy ou encore un cycle benzénique condensé.

2. Composés selon la revendication 1, dans lesquels R représente l'hydrogène, le chlore, un méthyle

ou un méthoxy ou un cycle benzénique condensé, et le second groupe amino occupe la position 6 ou 4.

3. Procédé de préparation des composés selon la revendication 1, caractérisé en ce que l'on réduit une dinitrophénylurée de formule générale

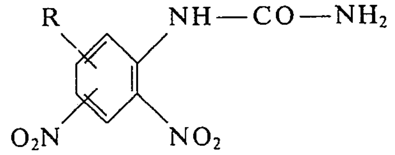

4. Procédé selon la revendication 3, caractérisé en ce que la réduction se fait par hydrogénation catalytique.

5. Procédé selon les revendications 3 ou 4, caractérisé en ce que la réduction est effectuée en milieu aqueux.

6. Procédé selon les revendications 4 ou 5, caractérisé en ce que la réduction est effectuée à un pH d'au moins 6.

7. L'utilisation des composés selon la revendication 1, pour préparer les amino-benzimidazolones de formule

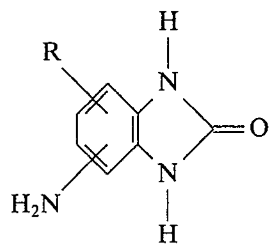

R ayant la même signification que dans la revendication 1, par cyclisation avec élimination d'ammoniac.

8. L'utilisation selon la revendication 7, caractérisée en ce que la cyclisation a lieu en milieu aqueux.

9. Utilisation selon la revendication 7 ou 8, caractérisée en ce que la cyclisation est effectuée à un pH de 1 à 10.

10. Utilisation selon l'une quelconque des revendications 7 à 9, caractérisée en ce que la cyclisation est effectuée sur un produit obtenu conformément à la revendication 3, sans isolement préalable de ce produit.